# EUROPEAN PATENT SPECIFICATION

(11) **EP 4 411 935 B1**
(45) Date of publication and mention of the grant of the patent: **29.04.2026**
(21) Application number: 23206934.4
(22) Date of filing: 31.10.2023
(51) Int. Cl.: H01M 10/54, B01D 5/00, H01M 4/86, C22B 7/00, B01D 1/00, H01M 10/0525

(54) **METHOD AND APPARATUS FOR RECOVERING ORGANIC CARBONATES**
VERFAHREN UND VORRICHTUNG ZUR RÜCKGEWINNUNG VON ORGANISCHEN CARBONATEN
PROCÉDÉ ET APPAREIL DE RÉCUPÉRATION DE CARBONATES ORGANIQUES

(30) Priority: 14.12.2022 JP 2022199559
(43) Date of publication of application: 07.08.2024
(73) Proprietor: TOYOTA JIDOSHA KABUSHIKI KAISHA, Toyota-shi, Aichi-ken, 471-8571 (JP)
(72) Inventor: MURAMATSU, Kenichiro, Toyota-shi, 471-8571 (JP); NORITAKE, Kazuki, Toyota-shi, 471-8571 (JP); ISOMURA, Keisuke, Toyota-shi, 471-8571 (JP); KASAHARA, Hiroshi, Toyota-shi, 471-8571 (JP)
(74) Representative: D Young & Co LLP

(56) References cited:
- CN-A- 111 573 953
- CN-A- 113 555 616
- US-A1- 2018 301 769
- US-A1- 2019 260 101
- US-A1- 2021 384 562

## Description

### BACKGROUND OF THE INVENTION

### 1. Field of the Invention

The disclosure relates to a method and an apparatus for recovering organic carbonates that are main components of an electrolytic solution of a waste lithium ion battery.

### 2. Description of Related Art

Japanese Unexamined Patent Application Publication No. 11-167936 (JP 11-167936 A) describes a method for recovering diethyl carbonate that is a main component of an electrolytic solution included in a spent lithium ion battery. In this existing method, a spent lithium ion battery is crushed in a freezing room, and the obtained crushed pieces are heated in diethyl carbonate serving as an extractant. This extraction is performed under a temperature condition lower than 25°C and higher than -43°C. The extractant is separated from a solid content of the crushed pieces. The diethyl carbonate contained in the electrolytic solution is recovered together with diethyl carbonate serving as an extractant by distillation of the extractant separated.

Other than JP 11-167936 A, Japanese Unexamined Patent Application Publication No. 2012-059564 (JP 2012-059564 A) is listed as a literature that describes a technology level in the technical field related to the disclosure. US2019260101, US2018301769 disclose a method for recovering organic carbonates contained in an electrolytic solution of a waste lithium ion battery.

CN111573953 discloses a method for recovering organic carbonate.

### SUMMARY OF THE INVENTION

In addition to diethyl carbonate (DEC), organic carbonates, such as ethylene carbonate (EC), dimethyl carbonate (DMC), and ethyl methyl carbonate (EMC), are used as main components of the electrolytic solution of a lithium ion battery. In the existing method, DEC is used as an extractant, so it is presumably suitable for recovery of DEC. However, on the other hand, recovery of other organic carbonates, such as EC, can be insufficient. The state of extracting DEC at the time of being brought into contact with crushed pieces is a liquid state. For this reason, extracting DEC adhering to the solid content of crushed pieces may be not able to be separated from the solid content, with the result that the recovery rate of DEC that is the main component of the electrolytic solution can be low.

The disclosure provides a technology capable of efficiently recovering multiple types of organic carbonates contained in an electrolytic solution of a lithium ion battery.

A first aspect of the disclosure provides a method for recovering organic carbonates contained in an electrolytic solution of a waste lithium ion battery according to claim 1. The organic carbonates include a first carbonate and a second carbonate having a melting point being higher than the first carbonate and a boiling point being higher than the first carbonate. The method includes performing a temperature adjustment process in which an internal temperature of a drier in which the waste lithium ion battery is accommodated is kept in a first temperature range, an internal temperature of a pipe extending to below the drier is kept in a second temperature range, and an internal temperature of a condenser connected to the drier via the pipe is kept in a third temperature range, and performing a first recovery process of reducing an internal pressure of the drier, an internal pressure of the pipe, and an internal pressure of the condenser from a normal pressure to a first pressure and recovering the first carbonate in the condenser. The first temperature range includes a temperature range in which only the first carbonate vaporizes at the first pressure and both the first carbonate and the second carbonate vaporize at a second pressure lower than the first pressure. The second temperature range includes a temperature range higher than or equal to the melting point of the second carbonate at the first pressure and lower than or equal to a thermal decomposition temperature of an electrolyte of the waste lithium ion battery at the second pressure. The third temperature range includes a temperature range in which the first carbonate liquefies at the first pressure.

In the first aspect, the third temperature range may include a temperature range in which the first carbonate liquefies at the first pressure, the second carbonate liquefies at the second pressure, and the first carbonate condensed at the first pressure does not vaporize again. In this case, the method may further include performing a second recovery process of, after the first recovery process of the first carbonate, reducing the internal pressure of the drier, the internal pressure of the pipe, and the internal pressure of the condenser from the first pressure to the second pressure and recovering the second carbonate in the condenser.

In the above configuration, the condenser may include a first condenser configured to recover the first carbonate and a second condenser disposed above the first condenser and configured to recover the second carbonate. The first pipe may connect the drier with the first condenser via the second condenser and the second pipe. When the first recovery process of the first carbonate is performed, the internal temperature of the drier, the internal temperature of the second pipe, and the internal temperature of the second condenser may be kept in the first temperature range, the internal temperature of the first pipe may be kept in the second temperature range, and the internal temperature of the first condenser may be kept in the third temperature range in the temperature adjustment process. When the second recovery process of the second carbonate is performed, the internal temperature of the drier, the internal temperature of the first pipe, and the internal temperature of the first condenser may be kept in the first temperature range, the internal temperature of the second pipe may be kept in the second temperature range, and the internal temperature of the second condenser may be kept in the third temperature range in the temperature adjustment process.

In the above configuration, the condenser may include a first condenser configured to recover the first carbonate and a second condenser configured to recover the second carbonate. The pipe may include a first pipe that connects the drier with the first condenser and a second pipe that connects the drier with the second condenser. The first pipe may branch off halfway from the second pipe. The method may further include performing a switching process of switching a connection destination of the drier between the first condenser and the second condenser. When the first recovery process of the first carbonate is performed, the internal temperature of the first pipe and the internal temperature of the second pipe may be kept in the second temperature range and the internal temperature of the first condenser may be kept in the third temperature range in the temperature adjustment process, and the connection destination of the drier may be switched to the first condenser in the switching process. When the second recovery process of the second carbonate is performed, the internal temperature of the second pipe may be kept in the second temperature range and the internal temperature of the second condenser may be kept in the third temperature range in the temperature adjustment process, and the connection destination of the drier may be switched to the second condenser in the switching process.

A second aspect of the disclosure provides an apparatus for recovering organic carbonates contained in an electrolytic solution of a waste lithium ion battery according to claim 5. The apparatus includes a drier, a pipe, a condenser, a pressure pump, a drier heater, a pipe heater, and a condenser heater. A waste lithium ion battery is accommodated in the drier. The pipe extends to below the drier. The condenser is connected to the drier via the pipe. The pressure pump is configured to adjust an internal pressure of the drier, an internal pressure of the pipe, and an internal pressure of the condenser. The drier heater is configured to adjust an internal temperature of the drier. The pipe heater is configured to adjust an internal temperature of the pipe. The condenser heater is configured to adjust an internal temperature of the condenser. The organic carbonates include a first carbonate and a second carbonate having a melting point being higher than the first carbonate and a boiling point being higher than the first carbonate. When the first recovery process of the first carbonate is performed, the drier heater is configured to keep the internal temperature of the drier in a first temperature range, the pipe heater is configured to keep the internal temperature of the pipe in a second temperature range, and the condenser heater is configured to keep the internal temperature of the condenser in a third temperature range. In the first recovery process of the first carbonate, the pressure pump is configured to reduce the internal pressure of the drier, the internal pressure of the pipe, and the internal pressure of the condenser from a normal pressure to a first pressure. The first temperature range includes a temperature range in which only the first carbonate vaporizes at the first pressure and both the first carbonate and the second carbonate vaporize at a second pressure lower than the first pressure. The second temperature range includes a temperature range higher than or equal to the melting point of the second carbonate at the first pressure and lower than or equal to a thermal decomposition temperature of an electrolyte of the waste lithium ion battery at the second pressure. The third temperature range includes a temperature range in which the first carbonate liquefies at the first pressure.

In the above aspect, the pipe may have a shape such that the pipe does not point upward between the drier and the condenser.

In the second aspect, the third temperature range may include a temperature range in which the first carbonate liquefies at the first pressure, the second carbonate liquefies at the second pressure, and the first carbonate condensed at the first pressure does not vaporize again. The second recovery process of the second carbonate may be performed after the first recovery process of the first carbonate. In this case, in the second recovery process of the second carbonate, the pressure pump may be configured to reduce the internal pressure of the drier, the internal pressure of the pipe, and the internal pressure of the condenser from the first pressure to the second pressure.

In the above configuration, the condenser may include a first condenser configured to recover the first carbonate and a second condenser disposed above the first condenser and configured to recover the second carbonate. The condenser heater may include a first condenser heater configured to adjust the internal temperature of the first condenser and a second condenser heater configured to adjust the internal temperature of the second condenser. The pipe may include a first pipe and a second pipe. The first pipe may connect the drier with the first condenser via the second pipe and the second condenser. The pipe heater may include a first pipe heater configured to adjust the internal temperature of the first pipe and a second pipe heater configured to adjust the internal temperature of the second pipe. When the first recovery process of the first carbonate is performed, the drier heater may be configured to keep the internal temperature of the drier in the first temperature range, the second pipe heater may be configured to keep the internal temperature of the second pipe in the first temperature range, the second condenser heater may be configured to keep the internal temperature of the second condenser in the first temperature range, the first pipe heater may be configured to keep the internal temperature of the first pipe in the second temperature range, and the first condenser heater may be configured to keep the internal temperature of the first condenser in the third temperature range. When the second recovery process of the second carbonate is performed, the drier heater may be configured to keep the internal temperature of the drier in the first temperature range, the first pipe heater may be configured to keep the internal temperature of the first pipe in the first temperature range, the first condenser heater may be configured to keep the internal temperature of the first condenser in the first temperature range, the second pipe heater may be configured to keep the internal temperature of the second pipe in the second temperature range, and the second condenser heater may be configured to keep the internal temperature of the second condenser in the third temperature range.

In the above configuration, the first pipe may have a shape such that the first pipe does not point upward between the first condenser and the second condenser.

In the above configuration, the second pipe may have a shape such that the second pipe does not point upward between the drier and the second condenser.

In the above configuration, the condenser may include a first condenser configured to recover the first carbonate and a second condenser configured to recover the second carbonate. The condenser heater may include a first condenser heater configured to adjust the internal temperature of the first condenser and a second condenser heater configured to adjust the internal temperature of the second condenser. The pipe may include a first pipe that connects the drier with the first condenser and a second pipe that connects the drier with the second condenser. The first pipe may branch off halfway from the second pipe. The pipe heater may include a first pipe heater configured to adjust the internal temperature of the first pipe and a second pipe heater configured to adjust the internal temperature of the second pipe. The apparatus may further include a switching valve provided at a branch point at which the first pipe branches off from the second pipe and configured to switch a connection destination of the drier between the first condenser and the second condenser. When the first recovery process of the first carbonate is performed, the first pipe heater may be configured to keep the internal temperature of the first pipe and the internal temperature of the second pipe in the second temperature range, the first condenser heater may be configured to keep the internal temperature of the first condenser in the third temperature range, and the switching valve may be configured to switch the connection destination of the drier to the first condenser. When the second recovery process of the second carbonate is performed, the second pipe heater may be configured to keep the internal temperature of the second pipe in the second temperature range, the second condenser heater may be configured to keep the internal temperature of the second condenser in the third temperature range, and the switching valve may be configured to switch the connection destination of the drier to the second condenser.

According to the aspects of the disclosure, it is possible to recover a first carbonate contained in an electrolytic solution of a waste lithium ion battery, separately from a second carbonate contained in the electrolytic solution. Since the second recovery process of the second carbonate is performed after the first recovery process of the first carbonate, not only the first carbonate but also the second carbonate can be recovered.

### BRIEF DESCRIPTION OF THE DRAWINGS

Features, advantages, and technical and industrial significance of exemplary embodiments of the invention will be described below with reference to the accompanying drawings, in which like signs denote like elements, and wherein:
FIG. 1 is a diagram that shows an example of the configuration of a recovery apparatus according to a first embodiment;
FIG. 2 is a flowchart that shows an example of a process of a controller particularly related to the first embodiment;
FIG. 3 is a diagram that shows an example of the configuration of a recovery apparatus according to a second embodiment;
FIG. 4 is a flowchart that shows an example of a process of a controller particularly related to the second embodiment;
FIG. 5 is a diagram that shows an example of the configuration of a recovery apparatus according to a third embodiment; and
FIG. 6 is a flowchart that shows an example of a process of a controller particularly related to the third embodiment.

### DETAILED DESCRIPTION OF EMBODIMENTS

Hereinafter, embodiments of the disclosure will be described with reference to the accompanying drawings. Like reference signs denote the same or corresponding portions in the drawings, and the description thereof is simplified or omitted.

### 1. Outline of Embodiments

### 1-1. Organic Carbonates

As described above, main components of an electrolytic solution of a lithium ion battery include organic carbonates such as ethylene carbonate (EC), dimethyl carbonate (DMC), ethyl methyl carbonate (EMC), and diethyl carbonate (DEC). In the aspect of the disclosure, these organic carbonates are recovered from a spent lithium ion battery (hereinafter, also referred to as "waste lithium ion battery").

In the aspect of the disclosure, the melting points and the boiling points of the organic carbonates to be recovered at room temperature and normal pressure are shown in Table 1.

**Table 1**

| Organic Carbonate | EC | DMC | EMC | DEC |
|---|---|---|---|---|
| Melting Point (°C) | 36.4 | 4.6 | -14.5 | -43 |
| Boiling Point (°C) | 244 | 90.3 | 107 | 126 |

As is apparent from Table 1, EC has the highest boiling point and the highest melting point, of the organic carbonates to be recovered. Particularly, the boiling point of EC is higher by 100°C or more than the boiling point of DEC having the second highest boiling point. Focusing on this point, in the aspect of the disclosure, a recovery process of organic carbonates (that is, DMC, EMC, and DEC) other than EC and a recovery process of EC are performed separately. Hereinafter, organic carbonates other than EC are also referred to as "first carbonates" or "first CNs", and EC is also referred to as "second carbonate" or "second CN".

### 1-2. Outline of Recovery Process

In the aspect of the disclosure, the recovery processes of the first and second carbonates are performed under heating and decompression conditions. The reason why the recovery processes are performed under heating and decompression conditions is to vaporize the first and second carbonates in a short time. However, as is apparent from the boiling points, the second CN is more difficult to vaporize than the first CNs. Therefore, in the aspect of the disclosure, the degree of decompression at which the recovery process of the second CN is performed is set so as to be higher than the degree of decompression at which the recovery process of the first CNs is performed. For example, the recovery process of the first CNs is performed at a pressure around 50 hPa (for example, 45 hPa to 55 hPa), and the recovery process of the second CN is performed at a pressure lower than 3 hPa (for example, 1 hPa to 2 hPa). Hereinafter, the pressure around 50 hPa is also referred to as "first pressure", and the pressure lower than 3 hPa is also referred to as "second pressure".

In this way, in the aspect of the disclosure, the pressure at which the recovery process of the second CN is performed is lower than the pressure at the recovery process of the first CNs is performed. Therefore, in the aspect of the disclosure, the recovery process of the first CNs is performed before the recovery process of the second CN. When the recovery processes of the first and second carbonates are performed in this order, the first CNs can be recovered from a waste lithium ion battery in the recovery process of the first CNs, and the second CN can be recovered from the waste lithium ion battery, from which the first CNs are removed, in the recovery process of the second CN.

In the aspect of the disclosure, in order to recover only the first CNs in the recovery process of the first CNs (that is, in order not to recover the second CN in the recovery process of the first CNs), a temperature condition of a vaporization system and a temperature condition of a condensation system are set based on a pressure condition under which the recovery process of the first CNs is performed. The temperature condition of the vaporization system is set to a condition such that the first CN vaporizes and the first CN does not thermally decompose at a pressure at which the recovery process of the first CN is performed. On the other hand, the temperature condition of the condensation system is set to a condition under which the first CNs liquefy at a pressure at which the recovery process of the first CNs is performed.

In the aspect of the disclosure, in order to recover the second CN in the recovery process of the second CN, the temperature condition of the vaporization system and the temperature condition of the condensation system are set based on a pressure condition under which the recovery process of the second CN is performed. The temperature condition of the vaporization system is set to a condition under which the second CN vaporizes and the first CNs do not thermally decompose at a pressure at which the recovery process of the second CN is performed. The reason why the "condition under which the first CNs do not thermally decompose" is used is because it is more difficult for EC to thermally decompose than the organic carbonates other than EC and, therefore, it is possible to suppress thermal decomposition of the second CN when the temperature condition is set to the "condition under which the first CNs do not thermally decompose". On the other hand, the temperature condition of the condensation system is set to a condition under which the second CN liquefies at a pressure at which the recovery process of the second CN is performed.

Hereinafter, an example of the vaporization system, an example of the condensation system, an example of the temperature condition of the vaporization system, and an example of the temperature condition of the condensation system will be described with reference to an example of the configuration of an apparatus.

### 2. First Embodiment

### 2-1. Example of Configuration of Apparatus

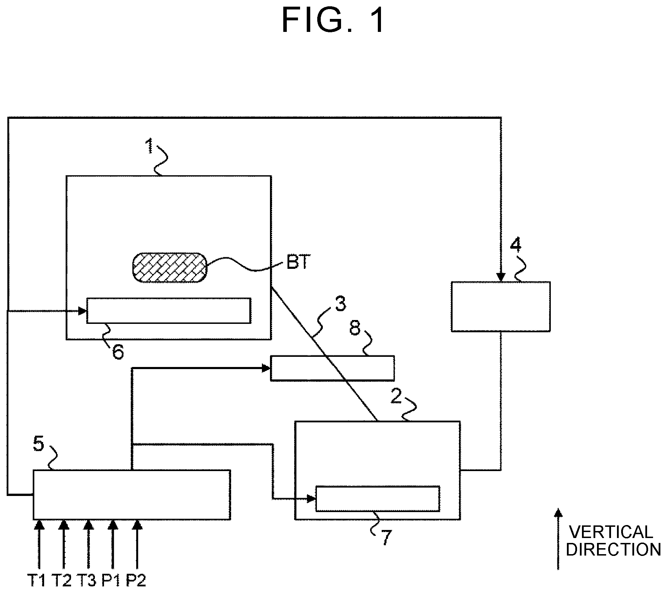

FIG. 1 is a diagram that shows an example of the configuration of a recovery apparatus according to a first embodiment of the disclosure. FIG. 1 shows an example of the configuration of the recovery apparatus that includes a drier 1, a condenser 2, a pipe 3, a pressure pump 4, and a controller 5. The drier 1 and the pipe 3 are components that may be regarded as the "vaporization system", and the condenser 2 is a component that may be regarded as the "condensation system". A heat insulating material (not shown) is provided at a connecting portion at which the drier 1 and the pipe 3 are connected, to reduce heat transfer from a constituent member of the drier 1 to the pipe 3. A heat insulating material (not shown) is also provided at a connecting portion at which the condenser 2 and the pipe 3 are connected.

Crushed pieces BT of a waste lithium ion battery are accommodated in the drier 1. The crushed pieces BT are obtained by, for example, crushing a waste lithium ion battery in a frozen state outside the recovery apparatus. Putting the crushed pieces BT into the drier 1 may be performed before heating of the drier 1 (described later) or may be performed after the heating. The drier 1 includes a drier heater 6 that adjusts the temperature of gas inside the drier 1.

The condenser 2 is a device for condensing first carbonates or a second carbonate in a gas state. The condenser 2 is disposed below the drier 1 in a vertical direction. The condenser 2 includes a condenser heater 7 that adjusts the temperature of gas inside the condenser 2. The condenser 2 is connected to the drier 1 via the pipe 3 having a straight shape. The pipe 3 includes a pipe heater 8 that adjusts the temperature of gas inside the pipe 3.

A connection port of the pipe 3 adjacent to the condenser 2 is located on the top surface of the condenser 2. On the other hand, a connection port of the pipe 3 adjacent to the drier 1 is located on the side surface of the drier 1. This is because the first carbonates or second carbonate vaporized in the drier 1 may liquefy halfway the pipe 3 under the heating and decompression conditions and, in this case, the first carbonates or second carbonate cannot move to the condenser 2.

In terms of this point, when the condenser 2 is disposed below the drier 1 and the pipe 3 that connects the drier 1 with the condenser 2 has a straight shape, it is possible to introduce the first carbonates or second carbonate vaporized in the drier 1 into the condenser 2. The shape of the pipe 3 is not necessarily straight. Any shape may be adopted as long as the shape of the pipe 3 does not point upward halfway. In other words, the pipe 3 has a shape such that the pipe 3 does not point upward between the drier 1 and the condenser 2.

The pressure pump 4 simultaneously adjusts the pressure of gas inside the drier 1, the pressure of gas inside the condenser 2, and the pressure of gas inside the pipe 3. The pressure pump 4 is a so-called pressure reducing pump. The pressure pump 4 operates in accordance with a control signal from the controller 5. When the pressure pump 4 operates, the pressure of gas inside the drier 1, the pressure of gas inside the condenser 2, and the pressure of gas inside the pipe 3 decrease. The pressure of gas inside the drier 1, the pressure of gas inside the condenser 2, and the pressure of gas inside the pipe 3, estimated as a result of operation of the pressure pump 4, each are a pressure around 50 hPa (that is, the first pressure) or a pressure lower than 3 hPa (that is, the second pressure).

The controller 5 is typically a computer including at least one processor and at least one memory. The controller 5 controls driving of the drier heater 6, the condenser heater 7, and the pipe heater 8. The controller 5 controls the operation of the pressure pump 4. To control the operations of these three types of heaters 6, 7, 8 and pressure pump 4, a temperature T1 and a pressure P1 of gas inside the drier 1, a temperature T2 and a pressure P2 of gas inside the condenser 2, and a temperature T3 of gas inside the pipe 3 are input to the controller 5. These physical quantities can be acquired from, for example, temperature sensors and pressure sensors respectively attached to the components of the drier 1 and the like.

The controller 5 controls the operations of the three types of heaters 6, 7, 8 based on the temperatures T1, T2, T3 such that the temperature of gas inside the drier 1 is kept in a "first temperature range", the temperature of gas inside the pipe 3 is kept in a "second temperature range", and the temperature of gas inside the condenser 2 is kept in a "third temperature range".

Here, the first temperature range is a temperature range in which only the first CNs vaporize (evaporate) at the first pressure and both the first and second CNs vaporize at the second pressure. The second temperature range is a temperature range higher than or equal to the melting point of the second CN at the first pressure and lower than or equal to a thermal decomposition temperature of an electrolyte (for example, LiPF₆) of the waste lithium ion battery at the second pressure. The third temperature range is a temperature range in which the first CNs liquefy (condense) at the first pressure, the second CN liquefies at the second pressure, and the first CNs condensed at the first pressure do not vaporize again. If the first and second CNs liquefied solidify, the first and second CNs clog in the condenser 2 or the pipe 3. To reduce such a situation, a lower limit of the third temperature range is desirably a temperature at which the first CNs condensed at the first pressure and the second CN condensed at the second pressure do not solidify. The first to third temperature ranges can be set in advance based on, for example, the first and second pressures and the melting points and the boiling points of organic carbonates to be recovered.

### 2-2. Example of Recovery of Organic Carbonates

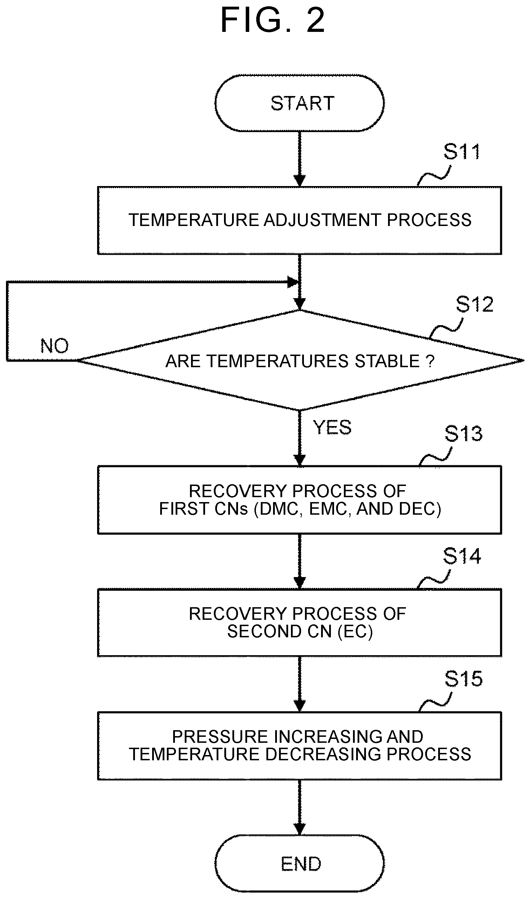

FIG. 2 is a flowchart that shows an example of a process of the controller 5 particularly related to the first embodiment. The flow of the process shown in FIG. 2 is executed, for example, after putting of crushed pieces BT into the drier 1 completes. Putting of crushed pieces BT into the drier 1 may be performed between step S12 and step S13. In this case, the flow of the process shown in FIG. 2 is executed, for example, as a result of startup of the recovery apparatus.

In the flow of the process shown in FIG. 2, initially, the controller 5 executes a temperature adjustment process (step S11). In the temperature adjustment process, for example, a heater operation control process is executed such that the temperature of gas inside the drier 1 is kept in the first temperature range, the temperature of gas inside the pipe 3 is kept in the second temperature range, and the temperature of gas inside the condenser 2 is kept in the third temperature range. A feedback control process based on the temperatures T1, T2, T3 is illustrated as the heater operation control process. The temperature adjustment process is continuously executed until the process of step S15 is started even after the process of step S11 completes.

Subsequently to the process of step S11, the controller 5 determines whether the temperatures are stable (step S12). When it is found that the temperatures T1, T2, T3 respectively fall within the target temperature ranges (that is, the first to third temperature ranges) as a result of the temperature adjustment process of step S11, the determination result of step S12 is affirmative. The process of step S12 is repeatedly executed until the affirmative determination result is obtained.

When the affirmative determination result is obtained in step S12, the recovery process of the first CNs (that is, DMC, EMC, and DEC) is performed (step S13). In the recovery process of the first CNs, the operation of the pressure pump 4 is controlled such that the pressure of gas inside the drier 1, the pressure of gas inside the condenser 2, and the pressure of gas inside the pipe 3 decrease from a normal pressure to a pressure around 50 hPa (that is, the first pressure). The recovery process of the first CNs is, for example, performed continuously for a period of time set in advance as a period of time that allows the first CNs to be sufficiently recovered based on the amount of crushed pieces BT put in the drier 1.

In a situation in which the temperature adjustment process is being executed, when the pressure of gas inside the drier 1, the pressure of gas inside the condenser 2, and the pressure of gas inside the pipe 3 decrease to the first pressure, the first CNs vaporize in the drier 1, and the first CNs flow into the condenser 2 through the pipe 3. The temperature of gas inside the pipe 3 is kept in the second temperature range. Therefore, part of the first CNs may liquefy in the pipe 3. In this case, the part of the first CNs flow into the condenser 2 in a liquid state. Then, the temperature of gas inside the condenser 2 is kept in the third temperature range. Therefore, the first CNs flowing into the condenser 2 in a gas state are cooled to liquefy here. Thus, the first CNs are recovered.

Subsequently to the process of step S13, the recovery process of the second CN (that is, EC) is performed (step S14). In the recovery process of the second CN, the operation of the pressure pump 4 is controlled such that the pressure of gas inside the drier 1, the pressure of gas inside the condenser 2, and the pressure of gas inside the pipe 3 decrease from the pressure around 50 hPa (that is, the first pressure) to the pressure lower than 3 hPa (that is, the second pressure). The recovery process of the second CN is, for example, performed continuously for a period of time set in advance as a period of time that allows the second CN to be sufficiently recovered based on the amount of crushed pieces BT put in the drier 1.

In a situation in which the temperature adjustment process is being executed, when the pressure of gas inside the drier 1, the pressure of gas inside the condenser 2, and the pressure of gas inside the pipe 3 decrease to the second pressure, the second CN vaporizes in the drier 1, and the second CN flows into the condenser 2 through the pipe 3. The temperature of gas inside the pipe 3 is kept in the second temperature range. Therefore, part of the second CN may liquefy in the pipe 3. In this case, the part of the second CN flows into the condenser 2 in a liquid state. The temperature of gas inside the condenser 2 is kept in the third temperature range. Therefore, the second CN flowing into the condenser 2 in a gas state is cooled to liquefy here. Thus, the second CN is recovered.

The first CNs and the second CN are desirably recovered with different condensers 2 such that the first CNs recovered in the recovery process of the first CNs and the second CN recovered in the recovery process of the second CN are not mixed in the condenser 2. This recovery example will be described in second and third embodiments.

Subsequently to the process of step S14, the controller 5 executes a pressure increasing and temperature decreasing process (step S15). The pressure increasing and temperature decreasing process is a process for returning the temperature and pressure of gas inside the drier 1, the temperature and pressure of gas inside the condenser 2, and the temperature and pressure of gas inside the pipe 3 to room temperature and normal pressure.

### 3. Second Embodiment

### 3-1. Example of Configuration of Apparatus

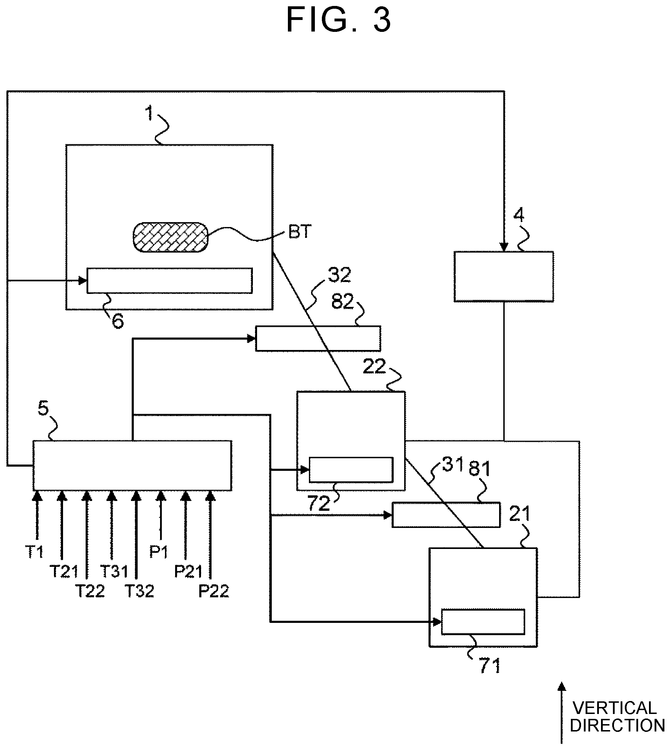

FIG. 3 is a diagram that shows an example of the configuration of a recovery apparatus according to a second embodiment of the disclosure. Different from the example of the configuration shown in FIG. 1, FIG. 3 shows an example of the configuration including two types of condensers. Specifically, FIG. 3 shows an example of the configuration of a recovery apparatus including a first condenser 21 and a second condenser 22, a first pipe 31 and a second pipe 32, a first condenser heater 71 and a second condenser heater 72, and a first pipe heater 81 and a second pipe heater 82.

The first condenser 21 is provided to recover the first CNs. The first condenser 21 is disposed below the second condenser 22. The first condenser heater 71 is provided to adjust the temperature of gas inside the first condenser 21. The first pipe 31 has a straight shape. The first pipe 31 connects the drier 1 with the first condenser 21 via the second pipe 32 and the second condenser 22. The first pipe 31 does not need to have a straight shape as long as the first pipe 31 has a shape such that the first pipe 31 does not point upward between the first condenser 21 and the second condenser 22.

A connection port of the first pipe 31 adjacent to the first condenser 21 is located on the top surface of the first condenser 21. On the other hand, a connection port of the first pipe 31 adjacent to the second condenser 22 is located on the side surface of the second condenser 22. The reason why such the shape and disposition of the first pipe 31 are adopted is basically the same as the reason why the shape and disposition of the pipe 3 described in the first embodiment.

The second condenser 22 is provided to recover the second CN. The second condenser heater 72 is provided to adjust the temperature of gas inside the second condenser 22. The second pipe 32 has a straight shape. The second pipe 32 connects the drier 1 with the second condenser 22. The second pipe 32 does not need to have a straight shape as long as the second pipe 32 has a shape such that the second pipe 32 does not point upward between the drier 1 and the second condenser 22.

A connection port of the second pipe 32 adjacent to the second condenser 22 is located on the top surface of the second condenser 22. On the other hand, a connection port of the second pipe 32 adjacent to the drier 1 is located on the side surface of the drier 1. The reason why such the shape and disposition of the second pipe 32 are adopted is basically the same as the reason why the shape and disposition of the pipe 3 described in the first embodiment.

In the second embodiment, the controller 5 controls the operations of the drier heater 6, the first condenser heater 71, the second condenser heater 72, the first pipe heater 81, and the second pipe heater 82. To control the operations of these five types of heaters 6, 71, 72, 81, 82, a temperature T1 and a pressure P1 of gas inside the drier 1, a temperature T21 and a pressure P21 of gas inside the first condenser 21, a temperature T22 and a pressure P22 of gas inside the second condenser 22, a temperature T31 of gas inside the first pipe 31, and a temperature T32 of gas inside the second pipe 32 are input to the controller 5.

When the controller 5 performs the recovery process of the first CNs, the controller 5 controls the operations of the five types of heaters 6, 71, 72, 81, 82 based on the temperatures T1, T21, T22, T31, T32 such that the temperature of gas inside the drier 1, the temperature of gas inside the second condenser 22, and the temperature of gas inside the second pipe 32 are kept in the first temperature range, the temperature of gas inside the first pipe 31 is kept in the second temperature range, and the temperature of gas inside the first condenser 21 is kept in the third temperature range.

On the other hand, when the controller 5 performs the recovery process of the second CN, the controller 5 controls the operations of three types of heaters 6, 72, 82 based on the temperatures T1, T22, T32 such that the temperature of gas inside the drier 1 is kept in the first temperature range, the temperature of gas inside the second pipe 32 is kept in the second temperature range, and the temperature of gas inside the second condenser 22 is kept in the third temperature range.

When the controller 5 performs the recovery process of the second CN, the controller 5 further controls the operations of the two types of heaters 71, 81 based on the temperatures T21, T31 such that the temperature of gas inside the first condenser 21 is kept in a temperature range higher than the third temperature range (for example, first temperature range) and the temperature of gas inside the first pipe 31 is kept in a temperature range higher than the second temperature range (for example, first temperature range). This is because liquefaction of the second CN in the middle of the first pipe 31 or in the first condenser 21 is suppressed to reliably recover the second CN in the second condenser 22.

Control of the controller 5 over the operation of the pressure pump 4 is the same as that of the first embodiment.

### 3-2. Example of Recovery of Organic Carbonates

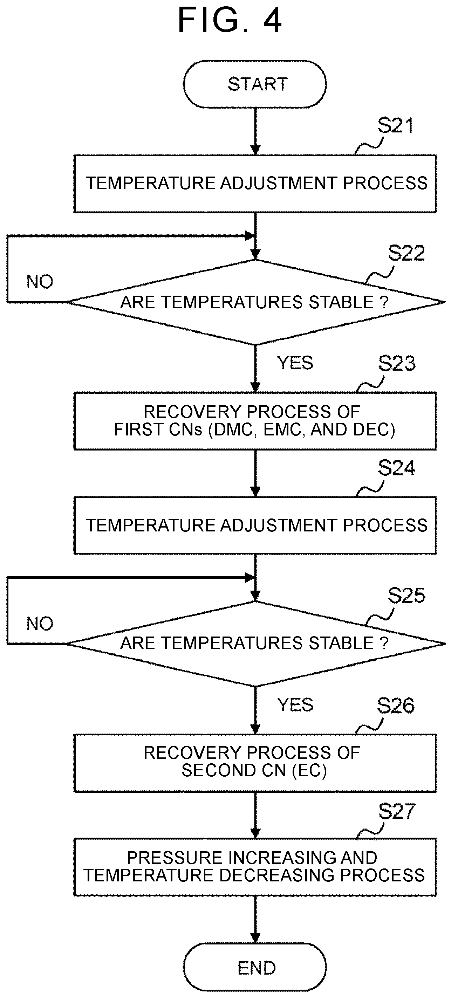

FIG. 4 is a flowchart that shows an example of a process of the controller 5 particularly related to the second embodiment. An example of the start timing of the flow of the process shown in FIG. 4 is the same as the flow of the process described with reference to FIG. 2.

In the flow of the process shown in FIG. 4, initially, the controller 5 executes a first temperature adjustment process (step S21). In the first temperature adjustment process, the controller 5 executes, for example, a heater operation control process such that the temperature of gas inside the drier 1, the temperature of gas inside the second condenser 22, and the temperature of gas inside the second pipe 32 are kept in the first temperature range, the temperature of gas inside the first pipe 31 is kept in the second temperature range, and the temperature of gas inside the first condenser 21 is kept in the third temperature range. A feedback control process based on the temperatures T1, T21, T22, T31, T32 is illustrated as the heater operation control process. The first temperature adjustment process is continuously executed until the process of step S24 is started even after the process of step S21 completes.

Subsequently to the process of step S21, the controller 5 determines whether the temperatures are stable (step S22). When it is found that the temperatures T1, T21, T22, T31, T32 respectively fall within the target temperature ranges (that is, the first to third temperature ranges) as a result of the first temperature adjustment process of step S21, the determination result of step S22 is affirmative. The process of step S22 is repeatedly executed until the affirmative determination result is obtained.

When the affirmative determination result is obtained in step S22, the recovery process of the first CNs (that is, DMC, EMC, and DEC) is performed (step S23). Details of the recovery process of the first CNs in step S23 are the same as those in step S13 of FIG. 2.

In a situation in which the first temperature adjustment process is being executed, when the pressure of gas inside each of the drier 1 and the like decreases to the first pressure, the first CNs vaporize in the drier 1, and the first CNs flow into the second condenser 22 through the second pipe 32. The first CNs in a gas state flowing into the second condenser 22 flow into the first condenser 21 through the first pipe 31. The reason why the first CNs keep a gas state in the second condenser 22 is because the temperature of gas inside the second condenser 22 and the temperature of gas inside the second pipe 32 are kept in the first temperature range.

On the other hand, the temperature of gas inside the first pipe 31 is kept in the second temperature range. Therefore, part of the first CNs flowing from the second condenser 22 into the first pipe 31 may liquefy here. In this case, the part of the first CNs flow into the first condenser 21 in a liquid state. The temperature of gas inside the first condenser 21 is kept in the third temperature range. Therefore, the first CNs flowing into the first condenser 21 in a gas state are cooled to liquefy here. Thus, the first CNs are recovered in the first condenser 21.

Subsequently to the process of step S23, the controller 5 executes a second temperature adjustment process (step S24). In the second temperature adjustment process, the controller 5 executes, for example, a heater operation control process such that the temperature of gas inside the drier 1, the temperature of gas inside the first condenser 21, and the temperature of gas inside the first pipe 31 are kept in the first temperature range, the temperature of gas inside the second pipe 32 is kept in the second temperature range, and the temperature of gas inside the second condenser 22 is kept in the third temperature range. A feedback control process based on the temperatures T1, T21, T22, T31, T32 is illustrated as the heater operation control process. The second temperature adjustment process is continuously executed until the process of step S27 is started even after the process of step S24 completes.

Subsequently to the process of step S24, the controller 5 determines whether the temperatures are stable (step S25). Details of the process of step S25 are basically the same as those of step S21. In other words, when it is found that the temperatures T1, T21, T22, T31, T32 respectively fall within the target temperature ranges (that is, the first to third temperature ranges) as a result of the second temperature adjustment process, the determination result of step S25 is affirmative. The process of step S25 is repeatedly executed until the affirmative determination result is obtained.

When the affirmative determination result is obtained in step S25, the recovery process of the second CN (that is, EC) is performed (step S26). Details of the recovery process of the second CN in step S26 are the same as those in step S14 of FIG. 2.

In a situation in which the second temperature adjustment process is being executed, when the pressure of gas inside each of the drier 1 and the like decreases to the second pressure, the second CN vaporizes in the drier 1, and the second CN flows into the second condenser 22 through the second pipe 32. The temperature of gas inside the second pipe 32 is kept in the second temperature range. Therefore, part of the second CN flowing from the drier 1 into the second pipe 32 may liquefy here. In this case, the part of the second CN flows into the second condenser 22 in a liquid state. The temperature of gas inside the second condenser 22 is kept in the third temperature range. Therefore, the second CN flowing into the second condenser 22 in a gas state is cooled to liquefy here. Thus, the second CN is recovered in the second condenser 22.

Subsequently to the process of step S26, the controller 5 executes a pressure increasing and temperature decreasing process (step S27). Details of the process of step S27 are the same as those of step S15 of FIG. 2.

### 4. Third Embodiment

### 4-1. Example of Configuration of Apparatus

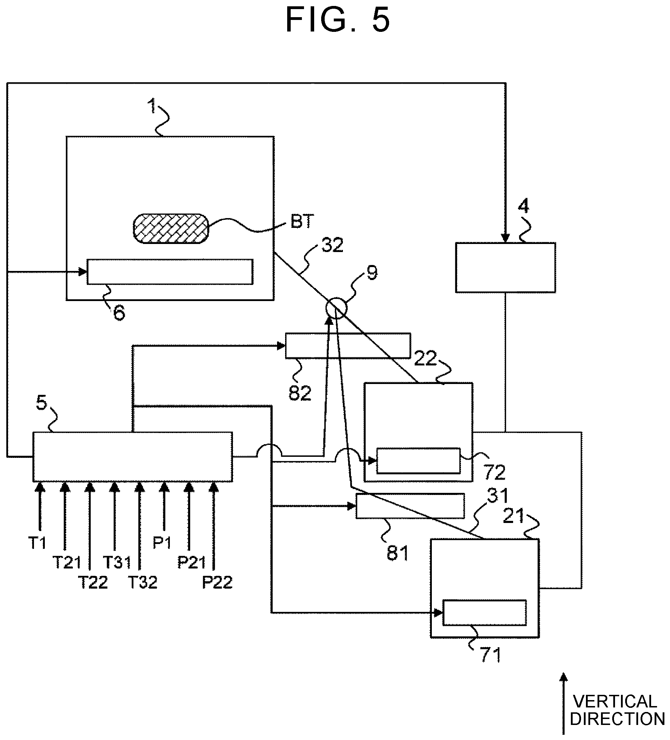

FIG. 5 is a diagram that shows an example of the configuration of a recovery apparatus according to a third embodiment of the disclosure. As in the case of the example of the configuration shown in FIG. 3, FIG. 5 shows an example of the configuration including two types of condensers. Specifically, the example of the configuration shown in FIG. 5 differs from the example of the configuration shown in FIG. 3 in the mode of connection among the drier 1, the first condenser 21, and the second condenser 22. In other words, in the example of the configuration shown in FIG. 3, the first condenser 21 and the second condenser 22 are connected to the drier 1 in series with each other. In contrast, in the example of the configuration shown in FIG. 5, the first condenser 21 and the second condenser 22 are connected to the drier 1 in parallel with each other.

Since the first condenser 21 and the second condenser 22 are connected in parallel with each other, the first pipe 31 branches halfway from the second pipe 32 in the example of the configuration shown in FIG. 5. A switching valve 9 is provided at a point at which the first pipe 31 branches off from the second pipe 32. The switching valve 9 switches a connection destination of the drier 1 between the first condenser 21 and the second condenser 22 based on a control signal from the controller 5.

In the example of the configuration shown in FIG. 3, the first condenser 21 is provided below the second condenser 22. In contrast, in the third embodiment, a location relationship between these condensers is not limited. In other words, a location relationship between the first condenser 21 and the second condenser 22 does not need to be the location relationship shown in FIG. 5. The location relationship may be inverted or the first condenser 21 and the second condenser 22 may be provided at the same height position. However, the first condenser 21 and the second condenser 22 are disposed below the drier 1 in the vertical direction.

In the third embodiment, the controller 5 controls the switching valve 9. Specifically, when the controller 5 performs the recovery process of the first CNs, the controller 5 switches the connection destination of the drier 1 to the first condenser 21. On the other hand, when the controller 5 performs the recovery process of the second CN, the controller 5 switches the connection destination of the drier 1 to the second condenser 22.

The controller 5 controls the operations of five types of heaters 6, 71, 72, 81, 82 based on the temperatures T1, T21, T22, T31, T32 such that the temperature of gas inside the drier 1 is kept in the first temperature range, the temperature of gas inside the first pipe 31 and the temperature of gas inside the second pipe 32 are kept in the second temperature range, and the temperature of gas inside the first condenser 21 and the temperature of gas inside the second condenser 22 are kept in the third temperature range.

Control of the controller 5 over the operation of the pressure pump 4 is the same as that of the first embodiment.

### 4-2. Example of Recovery of Organic Carbonates

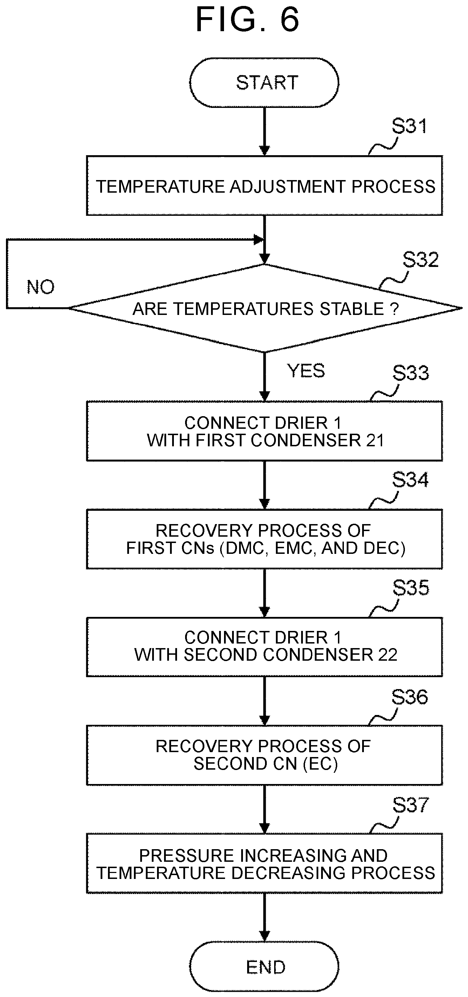

FIG. 6 is a flowchart that shows an example of a process of the controller 5 particularly related to the third embodiment. An example of the start timing of the flow of the process shown in FIG. 6 is the same as the flow of the process described with reference to FIG. 2.

In the flow of the process shown in FIG. 6, initially, the controller 5 executes a temperature adjustment process (step S31). In the temperature adjustment process, the controller 5 executes, for example, a heater operation control process such that the temperature of gas inside the drier 1 is kept in the first temperature range, the temperature of gas inside the first pipe 31 and the temperature of gas inside the second pipe 32 are kept in the second temperature range, and the temperature of gas inside the first condenser 21 and the temperature of gas inside the second condenser 22 are kept in the third temperature range. A feedback control process based on the temperatures T1, T21, T22, T31, T32 is illustrated as the heater operation control process. The temperature adjustment process is continuously executed until the process of step S37 is started even after the process of step S31 completes.

Subsequently to the process of step S31, the controller 5 determines whether the temperatures are stable (step S32). When it is found that the temperatures T1, T21, T22, T31, T32 respectively fall within the target temperature ranges (that is, the first to third temperature ranges) as a result of the temperature adjustment process of step S31, the determination result of step S32 is affirmative. The process of step S32 is repeatedly executed until the affirmative determination result is obtained.

When the affirmative determination result is obtained in step S32, the controller 5 controls the switching valve 9 such that the drier 1 and the first condenser 21 are connected (step S33). In other words, in the process of step S33, the connection destination of the drier 1 is switched to the first condenser 21.

Subsequently to the process of step S33, the recovery process of the first CNs (that is, DMC, EMC, and DEC) is performed (step S34). Details of the recovery process of the first CN in step S34 are the same as those in step S13 of FIG. 2.

In a situation in which the temperature adjustment process is being executed, when the pressure of gas inside the drier 1, the pressure of gas inside the first condenser 21, and the pressure of gas inside the first pipe 31 decrease to the first pressure, the first CNs vaporize in the drier 1, and the first CNs flow into the first condenser 21 through the first pipe 31. The temperature of gas inside the first pipe 31 and the temperature of gas inside the second pipe 32 are kept in the second temperature range. Therefore, part of the first CNs may liquefy in the second pipe 32 upstream of the installation location of the switching valve 9, or the first pipe 31. In this case, the part of the first CNs flow into the first condenser 21 in a liquid state. The temperature of gas inside the first condenser 21 is kept in the third temperature range. Therefore, the first CNs flowing into the first condenser 21 in a gas state are cooled to liquefy here. Thus, the first CNs are recovered in the first condenser 21.

Subsequently to the process of step S34, the controller 5 controls the switching valve 9 such that the drier 1 and the second condenser 22 are connected (step S35). In other words, in the process of step S35, the connection destination of the drier 1 is switched to the second condenser 22.

Subsequently to the process of step S35, the recovery process of the second CN (that is, EC) is performed (step S36). Details of the recovery process of the second CNs in step S36 are the same as those in step S14 of FIG. 2.

In a situation in which the temperature adjustment process is being executed, when the pressure of gas inside the drier 1, the pressure of gas inside the second condenser 22, and the pressure of gas inside the second pipe 32 decrease to the second pressure, the second CN vaporizes in the drier 1, and the second CN flows into the second condenser 22 through the second pipe 32. The temperature of gas inside the second pipe 32 is kept in the second temperature range. Therefore, part of the second CN may liquefy in the second pipe 32. In this case, the part of the second CN flows into the second condenser 22 in a liquid state. The temperature of gas inside the second condenser 22 is kept in the third temperature range. Therefore, the second CN flowing into the second condenser 22 in a gas state is cooled to liquefy here. Thus, the second CN is recovered in the second condenser 22.

Subsequently to the process of step S36, the controller 5 executes the pressure increasing and temperature decreasing process (step S37). Details of the process of step S37 are the same as those of step S15 of FIG. 2.

## Claims

1. A method for recovering organic carbonates contained in an electrolytic solution of a waste lithium ion battery,
the organic carbonates including a first carbonate and a second carbonate, the second carbonate having a melting point being higher than the first carbonate and a boiling point being higher than the first carbonate,
the method comprising:
performing a temperature adjustment process in which an internal temperature of a drier (1) in which the waste lithium ion battery is accommodated is kept in a first temperature range, an internal temperature of a pipe (3; 32) extending to below the drier (1) is kept in a second temperature range, and an internal temperature of a condenser (2) connected to the drier (1) via the pipe (3; 32) is kept in a third temperature range; and
performing a first recovery process of reducing an internal pressure of the drier (1), an internal pressure of the pipe (3; 32), and an internal pressure of the condenser (2) from a normal pressure to a first pressure and recovering the first carbonate in the condenser (2), wherein:
the first temperature range includes a temperature range in which only the first carbonate vaporizes at the first pressure and both the first carbonate and the second carbonate vaporize at a second pressure lower than the first pressure;
the second temperature range includes a temperature range higher than or equal to the melting point of the second carbonate at the first pressure and lower than or equal to a thermal decomposition temperature of an electrolyte of the waste lithium ion battery at the second pressure; and
the third temperature range includes a temperature range in which the first carbonate liquefies at the first pressure.

2. The method according to claim 1, wherein:
the third temperature range includes a temperature range in which the first carbonate liquefies at the first pressure, the second carbonate liquefies at the second pressure, and the first carbonate condensed at the first pressure does not vaporize again; and
the method further comprises performing a second recovery process of, after the first recovery process of the first carbonate, reducing the internal pressure of the drier (1), the internal pressure of the pipe (3), and the internal pressure of the condenser (2) from the first pressure to the second pressure and recovering the second carbonate in the condenser (2).

3. The method according to claim 2, wherein:
the condenser (2) includes a first condenser (21) configured to recover the first carbonate and a second condenser (22) disposed above the first condenser (21) and configured to recover the second carbonate;
the pipe (3) includes a first pipe (31) and a second pipe (32);
the first pipe (31) connects the drier (1) with the first condenser (21) via the second condenser (22) and the second pipe (32);
when the first recovery process of the first carbonate is performed, the internal temperature of the drier (1), the internal temperature of the second pipe (32), and the internal temperature of the second condenser (22) are kept in the first temperature range, the internal temperature of the first pipe (31) is kept in the second temperature range, and the internal temperature of the first condenser (21) is kept in the third temperature range in the temperature adjustment process; and
when the second recovery process of the second carbonate is performed, the internal temperature of the drier (1), the internal temperature of the first pipe (31), and the internal temperature of the first condenser (21) are kept in the first temperature range, the internal temperature of the second pipe (32) is kept in the second temperature range, and the internal temperature of the second condenser (22) is kept in the third temperature range in the temperature adjustment process.

4. The method according to claim 2, wherein:
the condenser (2) includes a first condenser (21) configured to recover the first carbonate and a second condenser (22) configured to recover the second carbonate;
the pipe (3) includes a first pipe (31) that connects the drier (1) with the first condenser (21) and a second pipe (32) that connects the drier (1) with the second condenser (22);
the first pipe (31) branches off halfway from the second pipe (32);
the method further comprises performing a switching process of switching a connection destination of the drier (1) between the first condenser (21) and the second condenser (22);
when the first recovery process of the first carbonate is performed, the internal temperature of the first pipe (31) and the internal temperature of the second pipe (32) are kept in the second temperature range and the internal temperature of the first condenser (21) is kept in the third temperature range in the temperature adjustment process, and the connection destination of the drier (1) is switched to the first condenser (21) in the switching process; and
when the second recovery process of the second carbonate is performed, the internal temperature of the second pipe (32) is kept in the second temperature range and the internal temperature of the second condenser (22) is kept in the third temperature range in the temperature adjustment process, and the connection destination of the drier (1) is switched to the second condenser (22) in the switching process.

5. An apparatus for recovering organic carbonates contained in an electrolytic solution of a waste lithium ion battery, the apparatus comprising:
a drier (1) in which a waste lithium ion battery is accommodated;
a pipe (3) extending to below the drier (1);
a condenser (2) connected to the drier (1) via the pipe (3);
a pressure pump (4) configured to adjust an internal pressure of the drier (1), an internal pressure of the pipe (3), and an internal pressure of the condenser (2);
a drier heater (6) configured to adjust an internal temperature of the drier (1);
a pipe heater (8) configured to adjust an internal temperature of the pipe (3); and
a condenser heater (7) configured to adjust an internal temperature of the condenser (2), wherein:
the organic carbonates include a first carbonate and a second carbonate, the second carbonate having a melting point being higher than the first carbonate and a boiling point being higher than the first carbonate;
when a first recovery process of the first carbonate is performed, the drier heater (6) is configured to keep the internal temperature of the drier (1) in a first temperature range, the pipe heater (8) is configured to keep the internal temperature of the pipe (3) in a second temperature range, and the condenser heater (7) is configured to keep the internal temperature of the condenser (2) in a third temperature range;
in the first recovery process of the first carbonate, the pressure pump (4) is configured to reduce the internal pressure of the drier (1), the internal pressure of the pipe (3), and the internal pressure of the condenser (2) from a normal pressure to a first pressure;
the first temperature range includes a temperature range in which only the first carbonate vaporizes at the first pressure and both the first carbonate and the second carbonate vaporize at a second pressure lower than the first pressure;
the second temperature range includes a temperature range higher than or equal to the melting point of the second carbonate at the first pressure and lower than or equal to a thermal decomposition temperature of an electrolyte of the waste lithium ion battery at the second pressure; and
the third temperature range includes a temperature range in which the first carbonate liquefies at the first pressure.

6. The apparatus according to claim 5, wherein the pipe (3) has a shape such that the pipe (3) does not face upward between the drier (1) and the condenser (2).

7. The apparatus according to claim 5 or 6, wherein:
the third temperature range includes a temperature range in which the first carbonate liquefies at the first pressure, the second carbonate liquefies at the second pressure, and the first carbonate condensed at the first pressure does not vaporize again;
a second recovery process of the second carbonate is performed after the first recovery process of the first carbonate; and
in the second recovery process of the second carbonate, the pressure pump (4) is configured to reduce the internal pressure of the drier (1), the internal pressure of the pipe (3), and the internal pressure of the condenser (2) from the first pressure to the second pressure.

8. The apparatus according to claim 7, wherein:
the condenser (2) includes a first condenser (21) configured to recover the first carbonate and a second condenser (22) disposed above the first condenser (21) and configured to recover the second carbonate;
the condenser heater (7) includes a first condenser heater (71) configured to adjust the internal temperature of the first condenser (21) and a second condenser heater (72) configured to adjust the internal temperature of the second condenser (22);
the pipe (3) includes a first pipe (31) and a second pipe (32);
the first pipe (31) connects the drier (1) with the first condenser (21) via the second pipe (32) and the second condenser (22);
the pipe heater (8) includes a first pipe heater (81) configured to adjust the internal temperature of the first pipe (31) and a second pipe heater (82) configured to adjust the internal temperature of the second pipe (32);
when the first recovery process of the first carbonate is performed, the drier heater (6) is configured to keep the internal temperature of the drier (1) in the first temperature range, the second pipe heater (82) is configured to keep the internal temperature of the second pipe (32) in the first temperature range, the second condenser heater (72) is configured to keep the internal temperature of the second condenser (22) in the first temperature range, the first pipe heater (81) is configured to keep the internal temperature of the first pipe (31) in the second temperature range, and the first condenser heater (71) is configured to keep the internal temperature of the first condenser (21) in the third temperature range; and
when the second recovery process of the second carbonate is performed, the drier heater (6) is configured to keep the internal temperature of the drier (1) in the first temperature range, the first pipe heater (81) is configured to keep the internal temperature of the first pipe (31) in the first temperature range, the first condenser heater (71) is configured to keep the internal temperature of the first condenser (21) in the first temperature range, the second pipe heater (82) is configured to keep the internal temperature of the second pipe (32) in the second temperature range, and the second condenser heater (72) is configured to keep the internal temperature of the second condenser (22) in the third temperature range.

9. The apparatus according to claim 8, wherein the first pipe (31) has a shape such that the first pipe (31) does not point upward between the first condenser (21) and the second condenser (22).

10. The apparatus according to claim 8 or 9, wherein the second pipe (32) has a shape such that the second pipe (32) does not point upward between the drier (1) and the second condenser (22).

11. The apparatus according to claim 7, wherein:
the condenser (2) includes a first condenser (21) configured to recover the first carbonate and a second condenser (22) configured to recover the second carbonate;
the condenser heater (7) includes a first condenser heater (71) configured to adjust the internal temperature of the first condenser (21) and a second condenser heater (72) configured to adjust the internal temperature of the second condenser (22);
the pipe (3) includes a first pipe (31) that connects the drier (1) with the first condenser (21) and a second pipe (32) that connects the drier (1) with the second condenser (22);
the first pipe (31) branches off halfway from the second pipe (32);
the pipe heater (8) includes a first pipe heater (81) configured to adjust the internal temperature of the first pipe (31) and a second pipe heater (82) configured to adjust the internal temperature of the second pipe (32);
the apparatus further comprises a switching valve (9) provided at a branch point at which the first pipe (31) branches off from the second pipe (32) and configured to switch a connection destination of the drier (1) between the first condenser (21) and the second condenser (22);
when the first recovery process of the first carbonate is performed, the first pipe heater (81) is configured to keep the internal temperature of the first pipe (31) and the internal temperature of the second pipe (32) in the second temperature range, the first condenser heater (71) is configured to keep the internal temperature of the first condenser (21) in the third temperature range, and the switching valve (9) is configured to switch the connection destination of the drier (1) to the first condenser (21); and
when the second recovery process of the second carbonate is performed, the second pipe heater (82) is configured to keep the internal temperature of the second pipe (32) in the second temperature range, the second condenser heater (72) is configured to keep the internal temperature of the second condenser (22) in the third temperature range, and the switching valve (9) is configured to switch the connection destination of the drier (1) to the second condenser (22).

## Patentansprüche

1. Verfahren zur Rückgewinnung organischer Carbonate, die in einer elektrolytischen Lösung einer Lithium-Ionen-Abfallbatterie enthalten sind,
wobei die organischen Carbonate ein erstes Carbonat und ein zweites Carbonat beinhalten, wobei das zweite Carbonat einen Schmelzpunkt aufweist, der höher ist als der des ersten Carbonats, und einen Siedepunkt aufweist, der höher ist als der des ersten Carbonats,
wobei das Verfahren Folgendes umfasst:
Durchführen eines Temperatureinstellungsprozesses, bei dem eine Innentemperatur eines Trockners (1), in dem die Lithium-Ionen-Abfallbatterie untergebracht ist, in einem ersten Temperaturbereich gehalten wird, eine Innentemperatur eines sich unter den Trockner (1) erstreckenden Rohrs (3; 32) in einem zweiten Temperaturbereich gehalten wird und eine Innentemperatur eines Kondensators (2), der über das Rohr (3; 32) mit dem Trockner (1) verbunden ist, in einem dritten Temperaturbereich gehalten wird; und
Durchführen eines ersten Rückgewinnungsprozesses zum Reduzieren eines Innendrucks des Trockners (1), eines Innendrucks des Rohrs (3; 32) und eines Innendrucks des Kondensators (2) von einem Normaldruck auf einen ersten Druck und Rückgewinnen des ersten Carbonats in dem Kondensator (2), wobei:
der erste Temperaturbereich einen Temperaturbereich beinhaltet, in dem nur das erste Carbonat bei dem ersten Druck verdampft und sowohl das erste Carbonat als auch das zweite Carbonat bei einem zweiten Druck verdampfen, der niedriger ist als der erste Druck;
der zweite Temperaturbereich einen Temperaturbereich beinhaltet, der höher als oder gleich dem Schmelzpunkt des zweiten Carbonats bei dem ersten Druck und niedriger als oder gleich einer Zersetzungstemperatur eines Elektrolyten der Lithium-Ionen-Abfallbatterie bei dem zweiten Druck ist; und
der dritte Temperaturbereich einen Temperaturbereich umfasst, in dem das erste Carbonat bei dem ersten Druck verflüssigt.

2. Verfahren nach Anspruch 1, wobei:
der dritte Temperaturbereich einen Temperaturbereich umfasst, in dem das erste Carbonat bei dem ersten Druck verflüssigt, das zweite Carbonat bei dem zweiten Druck verflüssigt und das bei dem ersten Druck kondensierte erste Carbonat nicht wieder verdampft; und
das Verfahren ferner Durchführen eines zweiten Rückgewinnungsprozesses, nach dem ersten Rückgewinnungsprozess des ersten Carbonats, Reduzieren des Innendrucks des Trockners (1), des Innendrucks des Rohrs (3) und des Innendrucks des Kondensators (2) vom ersten Druck auf den zweiten Druck und Rückgewinnen des zweiten Carbonats in dem Kondensator (2) umfasst.

3. Verfahren nach Anspruch 2, wobei:
der Kondensator (2) einen ersten Kondensator (21), der dazu ausgelegt ist, das erste Carbonat zurückzugewinnen, und einen zweiten Kondensator (22) beinhaltet, der über dem ersten Kondensator (21) angeordnet ist und dazu ausgelegt ist, das zweite Carbonat zurückzugewinnen;
das Rohr (3) ein erstes Rohr (31) und ein zweites Rohr (32) beinhaltet;
das erste Rohr (31) den Trockner (1) über den zweiten Kondensator (22) und das zweite Rohr (32) mit dem ersten Kondensator (21) verbindet;
wenn der erste Rückgewinnungsprozess des ersten Carbonats durchgeführt wird, die Innentemperatur des Trockners (1), die Innentemperatur des zweiten Rohrs (32) und die Innentemperatur des zweiten Kondensators (22) in dem ersten Temperaturbereich gehalten werden, die Innentemperatur des ersten Rohrs (31) in dem zweiten Temperaturbereich gehalten wird und die Innentemperatur des ersten Kondensators (21) bei dem Temperatureinstellungsprozess in dem dritten Temperaturbereich gehalten wird; und
wenn der zweite Rückgewinnungsprozess des zweiten Carbonats durchgeführt wird, die Innentemperatur des Trockners (1), die Innentemperatur des ersten Rohrs (31) und die Innentemperatur des ersten Kondensators (21) in dem ersten Temperaturbereich gehalten werden, die Innentemperatur des zweiten Rohrs (32) in dem zweiten Temperaturbereich gehalten wird und die Innentemperatur des zweiten Kondensators (22) bei dem Temperatureinstellungsprozess in dem dritten Temperaturbereich gehalten wird.

4. Verfahren nach Anspruch 2, wobei:
der Kondensator (2) einen ersten Kondensator (21), der dazu ausgelegt ist, das erste Carbonat zurückzugewinnen, und einen zweiten Kondensator (22) beinhaltet, der dazu ausgelegt ist, das zweite Carbonat zurückzugewinnen;
das Rohr (3) ein erstes Rohr (31), das den Trockner (1) mit dem ersten Kondensator (21) verbindet, und ein zweites Rohr (32) beinhaltet, das den Trockner (1) mit dem zweiten Kondensator (22) verbindet;
das erste Rohr (31) auf halber Strecke von dem zweiten Rohr (32) abzweigt;
das Verfahren ferner Durchführen eines Umschaltprozesses zum Umschalten eines Verbindungsziels des Trockners (1) zwischen dem ersten Kondensator (21) und dem zweiten Kondensator (22) umfasst;
wenn der erste Rückgewinnungsprozess des ersten Carbonats durchgeführt wird, die Innentemperatur des ersten Rohrs (31) und die Innentemperatur des zweiten Rohrs (32) im zweiten Temperaturbereich gehalten werden und die Innentemperatur des ersten Kondensators (21) bei dem Temperatureinstellungsprozess im dritten Temperaturbereich gehalten wird und das Verbindungsziel des Trockners (1) bei dem Umschaltprozess auf den ersten Kondensator (21) umgeschaltet wird; und
wenn der zweite Rückgewinnungsprozess des zweiten Carbonats durchgeführt wird, die Innentemperatur des zweiten Rohrs (32) im zweiten Temperaturbereich gehalten wird und die Innentemperatur des zweiten Kondensators (22) bei dem Temperatureinstellungsprozess im dritten Temperaturbereich gehalten wird und das Verbindungsziel des Trockners (1) bei dem Umschaltprozess auf den zweiten Kondensator (22) umgeschaltet wird.

5. Vorrichtung zur Rückgewinnung organischer Carbonate, die in einer elektrolytischen Lösung einer Lithium-Ionen-Abfallbatterie enthalten sind, wobei die Vorrichtung Folgendes umfasst:
einen Trockner (1), in dem eine Lithium-Ionen-Abfallbatterie untergebracht ist;
ein Rohr (3), das sich bis unter den Trockner (1) erstreckt;
einen Kondensator (2), der über das Rohr (3) mit dem Trockner (1) verbunden ist;
eine Druckpumpe (4), die dazu ausgelegt ist, einen Innendruck des Trockners (1), einen Innendruck des Rohrs (3) und einen Innendruck des Kondensators (2) einzustellen;
eine Trocknerheizung (6), die dazu ausgelegt ist, eine Innentemperatur des Trockners (1) einzustellen;
eine Rohrheizung (8), die dazu ausgelegt ist, eine Innentemperatur des Rohrs (3) einzustellen; und
eine Kondensatorheizung (7), die dazu ausgelegt ist, eine Innentemperatur des Kondensators (2) einzustellen, wobei:
die organischen Carbonate ein erstes Carbonat und ein zweites Carbonat beinhalten, wobei das zweite Carbonat einen Schmelzpunkt, der höher ist als der des ersten Carbonats, und einen Siedepunkt aufweist, der höher ist als der des ersten Carbonats;
wenn ein erster Rückgewinnungsprozess des ersten Carbonats durchgeführt wird, die Trocknerheizung (6) dazu ausgelegt ist, die Innentemperatur des Trockners (1) in einem ersten Temperaturbereich zu halten, die Rohrheizung (8) dazu ausgelegt ist, die Innentemperatur des Rohrs (3) in einem zweiten Temperaturbereich zu halten, und die Kondensatorheizung (7) dazu ausgelegt ist, die Innentemperatur des Kondensators (2) in einem dritten Temperaturbereich zu halten;
in dem ersten Rückgewinnungsprozess des ersten Carbonats die Druckpumpe (4) dazu ausgelegt ist, den Innendruck des Trockners (1), den Innendruck des Rohrs (3) und den Innendruck des Kondensators (2) von einem Normaldruck auf einen ersten Druck zu reduzieren;
der erste Temperaturbereich einen Temperaturbereich beinhaltet, in dem nur das erste Carbonat bei dem ersten Druck verdampft und sowohl das erste Carbonat als auch das zweite Carbonat bei einem zweiten Druck verdampfen, der niedriger ist als der erste Druck;
der zweite Temperaturbereich einen Temperaturbereich beinhaltet, der höher als oder gleich dem Schmelzpunkt des zweiten Carbonats bei dem ersten Druck und niedriger als oder gleich einer Zersetzungstemperatur eines Elektrolyten der Lithium-Ionen-Abfallbatterie bei dem zweiten Druck ist; und
der dritte Temperaturbereich einen Temperaturbereich umfasst, in dem das erste Carbonat bei dem ersten Druck verflüssigt.

6. Vorrichtung nach Anspruch 5, wobei das Rohr (3) eine solche Form aufweist, dass das Rohr (3) zwischen dem Trockner (1) und dem Kondensator (2) nicht nach oben zeigt.

7. Vorrichtung nach Anspruch 5 oder 6, wobei:
der dritte Temperaturbereich einen Temperaturbereich beinhaltet, in dem das erste Carbonat bei dem ersten Druck verflüssigt, das zweite Carbonat bei dem zweiten Druck verflüssigt und das bei dem ersten Druck kondensierte erste Carbonat nicht wieder verdampft;
ein zweiter Rückgewinnungsprozess des zweiten Carbonats nach dem ersten Rückgewinnungsprozess des ersten Carbonats durchgeführt wird; und
in dem zweiten Rückgewinnungsprozess des zweiten Carbonats die Druckpumpe (4) dazu ausgelegt ist, den Innendruck des Trockners (1), den Innendruck des Rohrs (3) und den Innendruck des Kondensators (2) vom ersten Druck auf den zweiten Druck zu reduzieren.

8. Vorrichtung nach Anspruch 7, wobei:
der Kondensator (2) einen ersten Kondensator (21), der dazu ausgelegt ist, das erste Carbonat zurückzugewinnen, und einen zweiten Kondensator (22) beinhaltet, der über dem ersten Kondensator (21) angeordnet ist und dazu ausgelegt ist, das zweite Carbonat zurückzugewinnen;
die Kondensatorheizung (7) eine erste Kondensatorheizung (71), die dazu ausgelegt ist, die Innentemperatur des ersten Kondensators (21) einzustellen, und eine zweite Kondensatorheizung (72) beinhaltet, die dazu ausgelegt ist, die Innentemperatur des zweiten Kondensators (22) einzustellen;
das Rohr (3) ein erstes Rohr (31) und ein zweites Rohr (32) beinhaltet;
das erste Rohr (31) den Trockner (1) über das zweite Rohr (32) und den zweiten Kondensator (22) mit dem ersten Kondensator (21) verbindet;
die Rohrheizung (8) eine erste Rohrheizung (81), die dazu ausgelegt ist, die Innentemperatur des ersten Rohrs (31) einzustellen, und eine zweite Rohrheizung (82) beinhaltet, die dazu ausgelegt ist, die Innentemperatur des zweiten Rohrs (32) einzustellen;
wenn der erste Rückgewinnungsprozess des ersten Carbonats durchgeführt wird, die Trocknerheizung (6) dazu ausgelegt ist, die Innentemperatur des Trockners (1) in dem ersten Temperaturbereich zu halten, die zweite Rohrheizung (82) dazu ausgelegt ist, die Innentemperatur des zweiten Rohrs (32) in dem ersten Temperaturbereich zu halten, die zweite Kondensatorheizung (72) dazu ausgelegt ist, die Innentemperatur des zweiten Kondensators (22) in dem ersten Temperaturbereich zu halten, die erste Rohrheizung (81) dazu ausgelegt ist, die Innentemperatur des ersten Rohrs (31) in dem zweiten Temperaturbereich zu halten, und die erste Kondensatorheizung (71) dazu ausgelegt ist, die Innentemperatur des ersten Kondensators (21) in dem dritten Temperaturbereich zu halten; und
wenn der zweite Rückgewinnungsprozess des zweiten Carbonats durchgeführt wird, die Trocknerheizung (6) dazu ausgelegt ist, die Innentemperatur des Trockners (1) in dem ersten Temperaturbereich zu halten, die erste Rohrheizung (81) dazu ausgelegt ist, die Innentemperatur des ersten Rohrs (31) in dem ersten Temperaturbereich zu halten, die erste Kondensatorheizung (71) dazu ausgelegt ist, die Innentemperatur des ersten Kondensators (21) in dem ersten Temperaturbereich zu halten, die zweite Rohrheizung (82) dazu ausgelegt ist, die Innentemperatur des zweiten Rohrs (32) in dem zweiten Temperaturbereich zu halten, und die zweite Kondensatorheizung (72) dazu ausgelegt ist, die Innentemperatur des zweiten Kondensators (22) in dem dritten Temperaturbereich zu halten.

9. Vorrichtung nach Anspruch 8, wobei das erste Rohr (31) eine solche Form aufweist, dass das erste Rohr (31) zwischen dem ersten Kondensator (21) und dem zweiten Kondensator (22) nicht nach oben zeigt.

10. Vorrichtung nach Anspruch 8 oder 9, wobei das zweite Rohr (32) eine solche Form aufweist, dass das zweite Rohr (32) zwischen dem Trockner (1) und dem zweiten Kondensator (22) nicht nach oben zeigt.

11. Vorrichtung nach Anspruch 7, wobei:
der Kondensator (2) einen ersten Kondensator (21), der dazu ausgelegt ist, das erste Carbonat zurückzugewinnen, und einen zweiten Kondensator (22) beinhaltet, der dazu ausgelegt ist, das zweite Carbonat zurückzugewinnen;
die Kondensatorheizung (7) eine erste Kondensatorheizung (71), die dazu ausgelegt ist, die Innentemperatur des ersten Kondensators (21) einzustellen, und eine zweite Kondensatorheizung (72) beinhaltet, die dazu ausgelegt ist, die Innentemperatur des zweiten Kondensators (22) einzustellen;
das Rohr (3) ein erstes Rohr (31), das den Trockner (1) mit dem ersten Kondensator (21) verbindet, und ein zweites Rohr (32) beinhaltet, das den Trockner (1) mit dem zweiten Kondensator (22) verbindet;
das erste Rohr (31) auf halber Strecke von dem zweiten Rohr (32) abzweigt;
die Rohrheizung (8) eine erste Rohrheizung (81), die dazu ausgelegt ist, die Innentemperatur des ersten Rohrs (31) einzustellen, und eine zweite Rohrheizung (82) beinhaltet, die dazu ausgelegt ist, die Innentemperatur des zweiten Rohrs (32) einzustellen;
die Vorrichtung ferner ein Umschaltventil (9) umfasst, das an einem Verzweigungspunkt bereitgestellt ist, an dem das erste Rohr (31) von dem zweiten Rohr (32) abzweigt, und dazu ausgelegt ist, ein Verbindungsziel des Trockners (1) zwischen dem ersten Kondensator (21) und dem zweiten Kondensator (22) umzuschalten;
wenn der erste Rückgewinnungsprozess des ersten Carbonats durchgeführt wird, die erste Rohrheizung (81) dazu ausgelegt ist, die Innentemperatur des ersten Rohrs (31) und die Innentemperatur des zweiten Rohrs (32) in dem zweiten Temperaturbereich zu halten, die erste Kondensatorheizung (71) dazu ausgelegt ist, die Innentemperatur des ersten Kondensators (21) in dem dritten Temperaturbereich zu halten, und das Umschaltventil (9) dazu ausgelegt ist, das Verbindungsziel des Trockners (1) auf den ersten Kondensator (21) umzuschalten; und
wenn der zweite Rückgewinnungsprozess des zweiten Carbonats durchgeführt wird, die zweite Rohrheizung (82) dazu ausgelegt ist, die Innentemperatur des zweiten Rohrs (32) in dem zweiten Temperaturbereich zu halten, die zweite Kondensatorheizung (72) dazu ausgelegt ist, die Innentemperatur des zweiten Kondensators (22) in dem dritten Temperaturbereich zu halten, und das Umschaltventil (9) dazu ausgelegt ist, das Verbindungsziel des Trockners (1) auf den zweiten Kondensator (22) umzuschalten.

## Revendications

1. Procédé destiné à récupérer des carbonates organiques contenus dans une solution électrolytique d'une batterie lithium-ion usagée,
les carbonates organiques incluant un premier carbonate et un deuxième carbonate, le deuxième carbonate ayant un point de fusion supérieur à celui du premier carbonate et un point d'ébullition supérieur à celui du premier carbonate,
le procédé comprenant :
la réalisation d'un processus d'ajustement de température dans lequel une température interne d'un déshydrateur (1) dans lequel est accueillie la batterie lithium-ion usagée est maintenue dans une première plage de température, une température interne d'un tuyau (3 ; 32) s'étendant jusqu'en dessous du déshydrateur (1) est maintenue dans une deuxième plage de température, et une température interne d'un condenseur (2) raccordé au déshydrateur (1) par le biais du tuyau (3 ; 32) est maintenue dans une troisième plage de température ; et
la réalisation d'un premier processus de récupération consistant à réduire une pression interne du déshydrateur (1), une pression interne du tuyau (3 ; 32) et une pression interne du condenseur (2) d'une pression normale à une première pression et récupérer le premier carbonate dans le condenseur (2), dans lequel :
la première plage de température englobe une plage de température dans laquelle seul le premier carbonate se vaporise à la première pression et à la fois le premier carbonate et le deuxième carbonate se vaporisent à une deuxième pression inférieure à la première pression ;
la deuxième plage de température englobe une plage de température supérieure ou égale au point de fusion du deuxième carbonate à la première pression et inférieure ou égale à une température de décomposition thermique d'un électrolyte de la batterie lithium-ion usagée à la deuxième pression ; et
la troisième plage de température englobe une plage de température dans laquelle le premier carbonate se liquéfie à la première pression.

2. Procédé selon la revendication 1, dans lequel :
la troisième plage de température englobe une plage de température dans laquelle le premier carbonate se liquéfie à la première pression, le deuxième carbonate se liquéfie à la deuxième pression, et le premier carbonate condensé à la première pression ne se vaporise pas à nouveau ; et
le procédé comprend en outre la réalisation d'un deuxième processus de récupération consistant, après le premier processus de récupération du premier carbonate, à réduire la pression interne du déshydrateur (1), la pression interne du tuyau (3) et la pression interne du condenseur (2) de la première pression à la deuxième pression et à récupérer le deuxième carbonate dans le condenseur (2).

3. Procédé selon la revendication 2, dans lequel :
le condenseur (2) inclut un premier condenseur (21) conçu pour récupérer le premier carbonate et un deuxième condenseur (22) disposé au-dessus du premier condenseur (21) et conçu pour récupérer le deuxième carbonate ;
le tuyau (3) inclut un premier tuyau (31) et un deuxième tuyau (32) ;
le premier tuyau (31) raccorde le déshydrateur (1) au premier condenseur (21) par le biais du deuxième condenseur (22) et du deuxième tuyau (32) ;
lorsque le premier processus de récupération du premier carbonate est réalisé, la température interne du déshydrateur (1), la température interne du deuxième tuyau (32) et la température interne du deuxième condenseur (22) sont maintenues dans la première plage de température, la température interne du premier tuyau (31) est maintenue dans la deuxième plage de température, et la température interne du premier condenseur (21) est maintenue dans la troisième plage de température lors du processus d'ajustement de température ; et
lorsque le deuxième processus de récupération du deuxième carbonate est réalisé, la température interne du déshydrateur (1), la température interne du premier tuyau (31) et la température interne du premier condenseur (21) sont maintenues dans la première plage de température, la température interne du deuxième tuyau (32) est maintenue dans la deuxième plage de température, et la température interne du deuxième condenseur (22) est maintenue dans la troisième plage de température lors du processus d'ajustement de température.

4. Procédé selon la revendication 2, dans lequel :
le condenseur (2) inclut un premier condenseur (21) conçu pour récupérer le premier carbonate et un deuxième condenseur (22) conçu pour récupérer le deuxième carbonate ;
le tuyau (3) inclut un premier tuyau (31) qui raccorde le déshydrateur (1) au premier condenseur (21) et un deuxième tuyau (32) qui raccorde le déshydrateur (1) au deuxième condenseur (22) ;
le premier tuyau (31) se ramifie à partir du deuxième tuyau (32) à mi-chemin de ce dernier ;
le procédé comprend en outre la réalisation d'un processus de commutation consistant à commuter une destination de raccordement du déshydrateur (1) entre le premier condenseur (21) et le deuxième condenseur (22) ; lorsque le premier processus de récupération du premier carbonate est réalisé, la température interne du premier tuyau (31) et la température interne du deuxième tuyau (32) sont maintenues dans la deuxième plage de température et la température interne du premier condenseur (21) est maintenue dans la troisième plage de température lors du processus d'ajustement de température, et la destination de raccordement du déshydrateur (1) est commutée vers le premier condenseur (21) lors du processus de commutation ; et
lorsque le deuxième processus de récupération du deuxième carbonate est réalisé, la température interne du deuxième tuyau (32) est maintenue dans la deuxième plage de température et la température interne du deuxième condenseur (22) est maintenue dans la troisième plage de température lors du processus d'ajustement de température, et la destination de raccordement du déshydrateur (1) est commutée vers le deuxième condenseur (22) lors du processus de commutation.

5. Appareil destiné à récupérer des carbonates organiques contenus dans une solution électrolytique d'une batterie lithium-ion usagée, l'appareil comprenant :
un déshydrateur (1) dans lequel est accueillie une batterie lithium-ion usagée ;
un tuyau (3) s'étendant jusqu'en dessous du déshydrateur (1) ;
un condenseur (2) raccordé au déshydrateur (1) par le biais du tuyau (3) ;
une pompe de pression (4) conçue pour ajuster une pression interne du déshydrateur (1), une pression interne du tuyau (3), et une pression interne du condenseur (2) ;
un dispositif de chauffage de déshydrateur (6) conçu pour ajuster une température interne du déshydrateur (1) ;
un dispositif de chauffage de tuyau (8) conçu pour ajuster une température interne du tuyau (3) ; et
un dispositif de chauffage de condenseur (7) conçu pour ajuster une température interne du condenseur (2), dans lequel :
les carbonates organiques incluent un premier carbonate et un deuxième carbonate, le deuxième carbonate ayant un point de fusion supérieur à celui du premier carbonate et un point d'ébullition supérieur à celui du premier carbonate ;
lorsqu'un premier processus de récupération du premier carbonate est réalisé, le dispositif de chauffage de déshydrateur (6) est conçu pour maintenir la température interne du déshydrateur (1) dans une première plage de température, le dispositif de chauffage de tuyau (8) est conçu pour maintenir la température interne du tuyau (3) dans une deuxième plage de température, et le dispositif de chauffage de condenseur (7) est conçu pour maintenir la température interne du condenseur (2) dans une troisième plage de température ;
lors du premier processus de récupération du premier carbonate, la pompe de pression (4) est conçue pour réduire la pression interne du déshydrateur (1), la pression interne du tuyau (3) et la pression interne du condenseur (2) d'une pression normale à une première pression ;
la première plage de température englobe une plage de température dans laquelle seul le premier carbonate se vaporise à la première pression et à la fois le premier carbonate et le deuxième carbonate se vaporisent à une deuxième pression inférieure à la première pression ;
la deuxième plage de température englobe une plage de température supérieure ou égale au point de fusion du deuxième carbonate à la première pression et inférieure ou égale à une température de décomposition thermique d'un électrolyte de la batterie lithium-ion usagée à la deuxième pression ; et
la troisième plage de température englobe une plage de température dans laquelle le premier carbonate se liquéfie à la première pression.

6. Appareil selon la revendication 5, dans lequel le tuyau (3) a une forme telle que le tuyau (3) ne pointe pas vers le haut entre le déshydrateur (1) et le condenseur (2).

7. Appareil selon la revendication 5 ou 6, dans lequel :
la troisième plage de température englobe une plage de température dans laquelle le premier carbonate se liquéfie à la première pression, le deuxième carbonate se liquéfie à la deuxième pression, et le premier carbonate condensé à la première pression ne se vaporise pas à nouveau ;
un deuxième processus de récupération du deuxième carbonate est réalisé après le premier processus de récupération du premier carbonate ; et
lors du deuxième processus de récupération du deuxième carbonate, la pompe de pression (4) est conçue pour réduire la pression interne du déshydrateur (1), la pression interne du tuyau (3) et la pression interne du condenseur (2) de la première pression à la deuxième pression.

8. Appareil selon la revendication 7, dans lequel :
le condenseur (2) inclut un premier condenseur (21) conçu pour récupérer le premier carbonate et un deuxième condenseur (22) disposé au-dessus du premier condenseur (21) et conçu pour récupérer le deuxième carbonate ;
le dispositif de chauffage de condenseur (7) inclut un premier dispositif de chauffage de condenseur (71) conçu pour ajuster la température interne du premier condenseur (21) et un deuxième dispositif de chauffage de condenseur (72) conçu pour ajuster la température interne du deuxième condenseur (22) ;
le tuyau (3) inclut un premier tuyau (31) et un deuxième tuyau (32) ;
le premier tuyau (31) raccorde le déshydrateur (1) au premier condenseur (21) par le biais du deuxième tuyau (32) et du deuxième condenseur (22) ;
le dispositif de chauffage de tuyau (8) inclut un premier dispositif de chauffage de tuyau (81) conçu pour ajuster la température interne du premier tuyau (31) et un deuxième dispositif de chauffage de tuyau (82) conçu pour ajuster la température interne du deuxième tuyau (32) ;
lorsque le premier processus de récupération du premier carbonate est réalisé, le dispositif de chauffage de déshydrateur (6) est conçu pour maintenir la température interne du déshydrateur (1) dans la première plage de température, le deuxième dispositif de chauffage de tuyau (82) est conçu pour maintenir la température interne du deuxième tuyau (32) dans la première plage de température, le deuxième dispositif de chauffage de condenseur (72) est conçu pour maintenir la température interne du deuxième condenseur (22) dans la première plage de température, le premier dispositif de chauffage de tuyau (81) est conçu pour maintenir la température interne du premier tuyau (31) dans la deuxième plage de température, et le premier dispositif de chauffage de condenseur (71) est conçu pour maintenir la température interne du premier condenseur (21) dans la troisième plage de température ; et
lorsque le deuxième processus de récupération du deuxième carbonate est réalisé, le dispositif de chauffage de déshydrateur (6) est conçu pour maintenir la température interne du déshydrateur (1) dans la première plage de température, le premier dispositif de chauffage de tuyau (81) est conçu pour maintenir la température interne du premier tuyau (31) dans la première plage de température,
le premier dispositif de chauffage de condenseur (71) est conçu pour maintenir la température interne du premier condenseur (21) dans la première plage de température,
le deuxième dispositif de chauffage de tuyau (82) est conçu pour maintenir la température interne du deuxième tuyau (32) dans la deuxième plage de température, et le deuxième dispositif de chauffage de condenseur (72) est conçu pour maintenir la température interne du deuxième condenseur (22) dans la troisième plage de température.

9. Appareil selon la revendication 8, dans lequel le premier tuyau (31) a une forme telle que le premier tuyau (31) ne pointe pas vers le haut entre le premier condenseur (21) et le deuxième condenseur (22).

10. Appareil selon la revendication 8 ou 9, dans lequel le deuxième tuyau (32) a une forme telle que le deuxième tuyau (32) ne pointe pas vers le haut entre le déshydrateur (1) et le deuxième condenseur (22).

11. Appareil selon la revendication 7, dans lequel :
le condenseur (2) inclut un premier condenseur (21) conçu pour récupérer le premier carbonate et un deuxième condenseur (22) conçu pour récupérer le deuxième carbonate ;
le dispositif de chauffage de condenseur (7) inclut un premier dispositif de chauffage de condenseur (71) conçu pour ajuster la température interne du premier condenseur (21) et un deuxième dispositif de chauffage de condenseur (72) conçu pour ajuster la température interne du deuxième condenseur (22) ;
le tuyau (3) inclut un premier tuyau (31) qui raccorde le déshydrateur (1) au premier condenseur (21) et un deuxième tuyau (32) qui raccorde le déshydrateur (1) au deuxième condenseur (22) ;
le premier tuyau (31) se ramifie à partir du deuxième tuyau (32) à mi-chemin de ce dernier ;
le dispositif de chauffage de tuyau (8) inclut un premier dispositif de chauffage de tuyau (81) conçu pour ajuster la température interne du premier tuyau (31) et un deuxième dispositif de chauffage de tuyau (82) conçu pour ajuster la température interne du deuxième tuyau (32) ;
l'appareil comprend en outre une vanne de commutation (9) disposée à un point de ramification auquel le premier tuyau (31) se ramifie à partir du deuxième tuyau (32) et conçue pour commuter une destination de raccordement du déshydrateur (1) entre le premier condenseur (21) et le deuxième condenseur (22) ;
lorsque le premier processus de récupération du premier carbonate est réalisé, le premier dispositif de chauffage de tuyau (81) est conçu pour maintenir la température interne du premier tuyau (31) et la température interne du deuxième tuyau (32) dans la deuxième plage de température, le premier dispositif de chauffage de condenseur (71) est conçu pour maintenir la température interne du premier condenseur (21) dans la troisième plage de température, et la vanne de commutation (9) est conçue pour commuter la destination de raccordement du déshydrateur (1) vers le premier condenseur (21) ; et
lorsque le deuxième processus de récupération du deuxième carbonate est réalisé, le deuxième dispositif de chauffage de tuyau (82) est conçu pour maintenir la température interne du deuxième tuyau (32) dans la deuxième plage de température, le deuxième dispositif de chauffage de condenseur (72) est conçu pour maintenir la température interne du deuxième condenseur (22) dans la troisième plage de température, et la vanne de commutation (9) est conçue pour commuter la destination de raccordement du déshydrateur (1) vers le deuxième condenseur (22).
